# EUROPEAN PATENT APPLICATION

(11) **EP 4 588 434 A1**
(43) Date of publication of application: **23.07.2025**
(21) Application number: 23882931.1
(22) Date of filing: 27.09.2023
(51) Int. Cl.: A61B 5/024, G02B 1/10, G04G 21/02, G06F 1/16

(54) **ELECTRONIC DEVICE INCLUDING OPTICAL COATING LAYER DISPOSED ON COVER**

(30) Priority: 26.10.2022 KR 20220139719; 16.11.2022 KR 20220153555
(71) Applicant: Samsung Electronics Co., Ltd., Suwon-si, Gyeonggi-do 16677 (KR)
(72) Inventor: LEE, Jeahyuck, Suwon-si Gyeonggi-do 16677 (KR); KIM, Younghyun, Suwon-si Gyeonggi-do 16677 (KR); BAEK, Sanghyun, Suwon-si Gyeonggi-do 16677 (KR); YOO, Hyunguk, Suwon-si Gyeonggi-do 16677 (KR); JEONG, Injo, Suwon-si Gyeonggi-do 16677 (KR)
(74) Representative: Gulde & Partner
(86) International application number: PCT/KR2023/015021
(87) International publication number: WO 2024/090824

(57) **Abstract**

This electronic device may include: a cover including a transparent portion; an optical sensor that is spaced from the cover and includes a light-emitting portion configured to transmit first light portions to the outside through the transparent portion of the cover and at least one light-receiving portion spaced from the light-emitting portion, configured to receive second light portions through the transparent portion, and surrounding the outside of the light-emitting portion; a first optical coating disposed on one surface of the transparent portion and facing the light-emission portion; and a second optical coating spaced from the first optical coating, disposed on one surface of the transparent portion, and facing the at least one light-receiving portion. The first optical coating may include patterns that are concentric around a location overlapping the light-emitting portion. Other embodiments are also possible.

## Description

### [Technical Field]

The present disclosure relates to an electronic device including an optical coating layer disposed on a cover.

### [Background Art]

An electronic device may include sensors for obtaining information around the electronic device to provide various functions to a user. For example, the electronic device may include an optical sensor to obtain information on the user's body.

### [Disclosure]

### [Technical Solution]

According to an embodiment, an electronic device may include a cover forming at least a portion of an exterior of a housing and including a transparent portion. The electronic device may include an optical module that includes a light-emitting portion, spaced apart from the cover, configured to transmit first light to an outside through the transparent portion, and at least one light-receiving portion, spaced apart from the light-emitting portion, configured to receive second light through the transparent portion, and surrounding an outer periphery of the light-emitting portion. The electronic device may include a first optical coating disposed on a surface of the transparent portion facing the light-emitting portion. The electronic device may include a second optical coating, spaced apart from the first optical coating, disposed on a surface of the transparent portion facing the at least one light-receiving portion. The first optical coating may include concentric patterns with respect to a location overlapping the light-emitting portion.

According to an embodiment, an electronic device may include a cover forming at least a portion of an exterior of a housing and including a transparent portion. The electronic device may include an optical module that includes a light-emitting portion, spaced apart from the cover, configured to transmit first light to an outside through the transparent portion, at least one light-receiving portion, spaced apart from the light-emitting portion, configured to receive second light through the transparent portion, and surrounding an outer periphery of the light-emitting portion, and a partition wall disposed between the light-emitting portion and the light-receiving portion. The electronic device may include a first optical coating disposed on a surface of the transparent portion facing the light-emitting portion. The electronic device may include a second optical coating spaced apart from the first optical coating and disposed on a surface of the transparent portion facing the at least one light- emitting portion. The electronic device may include a light-blocking member disposed between the first optical coating and the second optical coating. According to an embodiment, the first optical coating may include a first pattern. The first optical coating may include a second pattern spaced apart from an outer periphery of the first pattern by a first distance, surrounding around an edge of the first pattern, and having a first width. The first optical coating may include a third pattern spaced apart from an outer periphery of the second pattern by a second distance, surrounding an edge of the second pattern, and having a second width.

According to an embodiment, the electronic device may include a cover forming at least a portion of an exterior of the electronic device and including a transparent portion. The electronic device may include an optical module that includes a light-emitting portion, at least one light-receiving portion, and a partition wall. The light-emitting portion may be configured to be spaced apart from the cover and transmit first light to the outside through the transparent portion. The at least one light-receiving portion may be configured to be spaced apart from the light-emitting portion and receive second light through the transparent portion, and may surround an outer periphery of the light-emitting portion. The partition wall may be disposed between the light-emitting portion and the at least one light-receiving portion. The electronic device may include a light-blocking material. The light-blocking material may include an optical coating disposed on a surface of the transparent portion facing the light-emitting portion and the light-receiving portion, and a light-blocking material disposed between a region of the optical coating overlapping the light-emitting portion and a region of the optical coating overlapping the light-receiving portion.

### [Description of the Drawings]

FIG. 1 is a block diagram of an electronic device in a network environment according to various embodiments.
FIG. 2A is a perspective view of a front surface of an electronic device according to an embodiment.
FIG. 2B is a perspective view of a rear surface of the electronic device of FIG. 2A.
FIG. 3 is an exploded perspective view of the electronic device of FIG. 2A.
FIG. 4 is an exploded perspective view illustrating a cover and an optical module of an electronic device according to an embodiment.
FIGS. 5A and 5B are schematic views illustrating an electronic device including a cover and an optical module in a state in which an electronic device is in contact with an external object, according to an embodiment.
FIG. 6 is a diagram illustrating a pattern of a first optical coating disposed in a region A of FIG. 4 of the cover according to an embodiment.
FIG. 7 is an enlarged view of a region B of FIG. 5A according to an embodiment.
FIG. 8 is an enlarged view of a region C of FIG. 5A or FIG. 5B, according to an embodiment.
FIG. 9 is a graph illustrating a relationship between an incident angle and transmittance of light incident on an optical coating according to an embodiment.
FIG. 10 is a diagram illustrating an exemplary pattern of a first optical coating according to an embodiment.

### [Mode for Invention]

Hereinafter, embodiments of the present disclosure will be described in detail with reference to the drawings so that those skilled in the art may easily implement the present disclosure. However, the present disclosure may be implemented in several different forms and is not limited to the embodiments described herein. With respect to the description of the drawing, the same or similar reference numerals may be used for the same or similar components. Additionally, descriptions of well-known functions and configurations may be omitted in the drawings and related descriptions for clarity and conciseness.

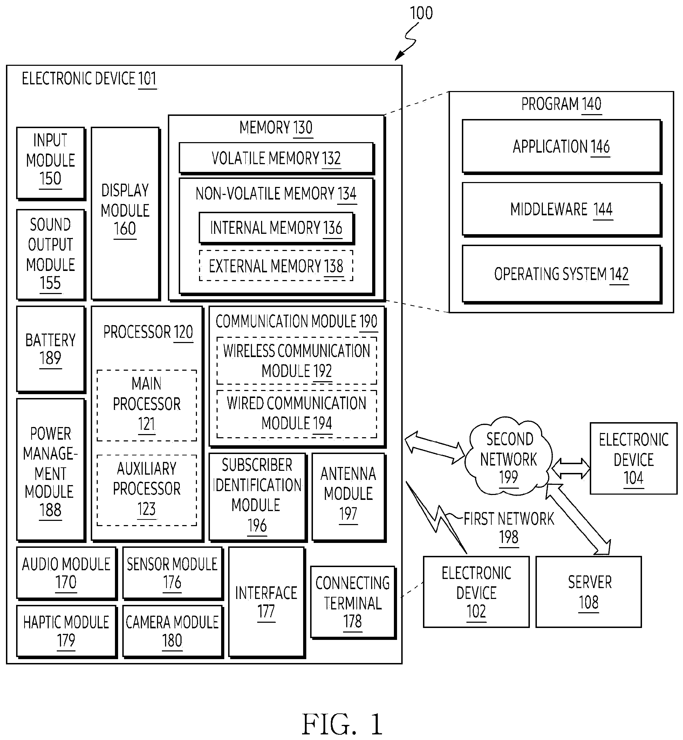

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

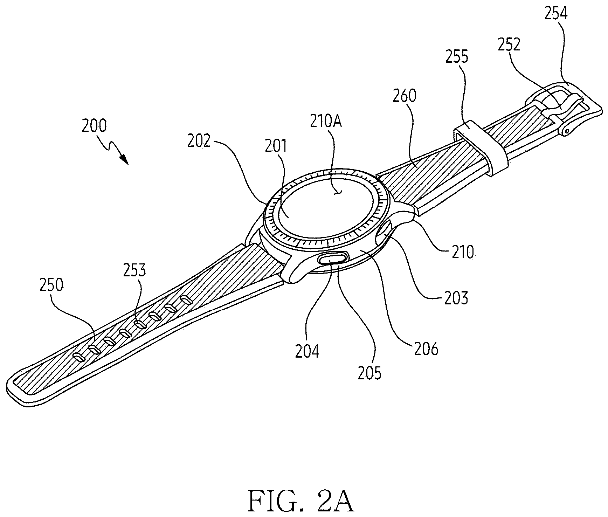

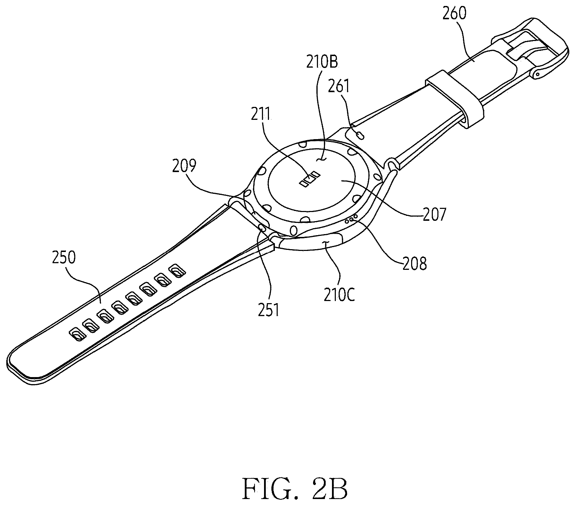

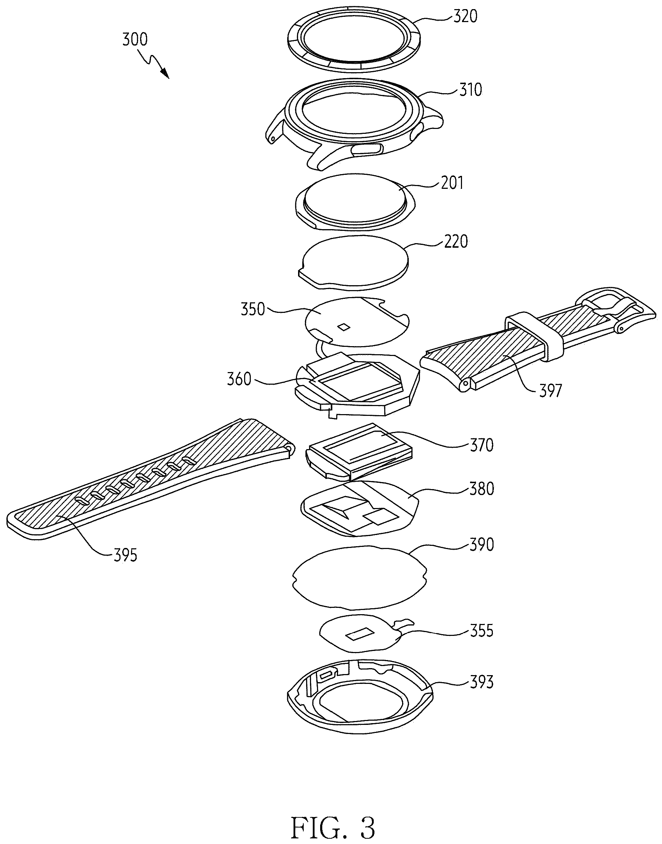

FIG. 2A is a perspective view of a front surface of an electronic device according to an embodiment. FIG. 2B is a perspective view of a rear surface of the electronic device of FIG. 2A. FIG. 3 is an exploded perspective view of the electronic device of FIG. 2A.

Referring to FIGS. 2A and 2B , an electronic device 200 according to an embodiment (e.g., the electronic device 101 of FIG. 1) may include a housing 210 including a first surface (or front surface) 210A, a second surface (or rear surface) 210B, and a side surface 210C surrounding a space between the first surface 210A and the second surface 210B, and fastening members 250 and 260 connected to at least a portion of the housing 210 and configured to detachably fasten the electronic device 200 to a portion of a user's body (e.g., a wrist or an ankle). In another embodiment (not illustrated), the housing may refer to a structure forming a portion of the first surface 210A, the second surface 210B, and the side surface 210C of FIG. 2A. According to an embodiment, the first surface 210A may be formed by a front plate 201 (e.g., a glass plate or a polymer plate including various coating layers) that is at least partially substantially transparent. The second surface 210B may be formed by a rear plate 207 that is substantially opaque. For example, the rear plate 207 may be formed of coated or colored glass, ceramic, polymer, metal (e.g., aluminum, stainless steel (STS), or magnesium), or a combination of at least two of the materials. The side surface 210C may be coupled to the front plate 201 and the rear plate 207 and may be formed by a side bezel structure (or "side member") 206 including metal and/or polymer. In some embodiments, the rear plate 207 and the side bezel structure 206 may be integrally formed and include the same material (e.g., a metallic material such as aluminum). The fastening members 250 and 260 may be formed of various materials and shapes. The integral and plurality of unit links may be formed of weaving, leather, rubber, urethane, metal, ceramic, or a combination of at least two of the materials to be movable with each other.

According to an embodiment, the electronic device 200 may include at least one of a display 220 (with reference FIG. 3), audio modules 205 and 208, a sensor module 211, key input devices 202, 203, and 204, and a connector hole 209. In some embodiments, the electronic device 200 may omit at least one (e.g., the key input devices 202, 203, and 204, the connector hole 209, or the sensor module 211) of the components or may additionally include another component.

For example, the display 220 may be exposed through a significant portion of the front plate 201. A shape of the display 220 may correspond to a shape of the front plate 201, and may have various shapes such as a circle, an oval, or a polygon. The display 220 may be coupled to or disposed adjacent to a touch sensing circuit, a pressure sensor capable of measuring intensity (pressure) of a touch, and/or a fingerprint sensor.

The audio modules 205 and 208 may include a microphone hole 205 and a speaker hole 208. A microphone for obtaining an external sound may be disposed inside the microphone hole 205, and in some embodiments, a plurality of microphones may be disposed to detect a direction of a sound. The speaker hole 208 may be used as an external speaker and a call receiver. In some embodiments, the speaker hole 208 and the microphone hole 203 may be implemented as a single hole, or a speaker may be included without the speaker hole 208 (e.g., a piezo speaker).

The sensor module 211 may generate an electrical signal or a data value corresponding to an operating state inside the electronic device 200 or an external environmental state. For example, the sensor module 211 may include a biometric sensor module 211 (e.g., a heart rate monitor (HRM) sensor) disposed on the second surface 210B of the housing 210. For example, the electronic device 200 may further include at least one of a sensor module, a gesture sensor, a gyro sensor, a barometric sensor, a magnetic sensor, an acceleration sensor, a grip sensor, a color sensor, an IR sensor, a biometric sensor, a temperature sensor, a humidity sensor, or an illuminance sensor, which are not illustrated.

The key input devices 202, 203, and 204 may include a wheel key 202 disposed on the first surface 210A of the housing 210 and rotatable in at least one direction, and side key buttons 202 and 203 disposed on the side surface 210C of the housing 210. The wheel key may have a shape corresponding to the shape of the front plate 201. In another embodiment, the electronic device 200 may not include some or all of the above-described key input devices 202, 203, and 204, and the excluded key input devices 202, 203, and 204 may be implemented in another form, such as a soft key, on the display 220. The connector hole 209 may accommodate a connector (e.g., a USB connector) for transmitting and receiving power and/or data with an external electronic device and include another connector hole (not illustrated) capable of accommodating a connector for transmitting and receiving an audio signal with an external electronic device. For example, the electronic device 200 may further include a connector cover (not illustrated) covering at least a portion of the connector hole 209 and blocking the inflow of external foreign substances into the connector hole.

The fastening members 250 and 260 may be detachably coupled to at least a portion of the housing 210 using locking members 251 and 261. The fastening members 250 and 260 may include one or more of a fixing member 252, a fixing member fastening hole 253, a band guide member 254, and a band fixing ring 255.

The fixing member 252 may be configured to fix the housing 210 and the fastening members 250 and 260 to a part (e.g., wrist or ankle) of the user's body. The fixing member fastening hole 253 may fix the housing 210 and the fastening members 250 and 260 to a part of the user's body corresponding to the fixing member 252. The band guide member 254 may be configured to limit a movement range of the fixing member 252 when the fixing member 252 is fastened to the fixing member fastening hole 253, so that the fastening members 250 and 260 are fasten in close contact with a part of the user's body.

Referring to FIG. 3, an electronic device 300 (e.g., the electronic device 101 of FIG. 1 or the electronic device 200 of FIG. 2A) may include a frame 310, a wheel key 320 (e.g., the wheel key 202 of FIG. 2A), a front plate 201, a display 220, a first antenna 350, a second antenna 355, a support member 360 (e.g., bracket), a battery 370, a PCB 380, a sealing member 390, a rear plate 393, and fastening members 395 and 397 (e.g., the fastening members 250 and 260 of FIGS. 2A to 2B). At least one of the components of the electronic device 300 may be the same as or similar to at least one of the components of the electronic device 200 of FIG. 2A or 2B, and a redundant description will be omitted hereinafter. The support member 360 may be disposed inside the electronic device 300 to be connected to the frame 310, or may be integrally formed with the frame 310. The frame 310 may be referred to as a side bezel structure in terms of determining a shape of a side surface of the electronic device 300. The support member 360 may be formed of, for example, a metal material and/or a non-metal material (e.g., polymer). In the support member 360, the display 220 may be coupled to a surface and the PCB 380 may be coupled to another surface. The PCB 380 may be mounted with a processor (e.g., the processor 120 of FIG. 1), memory (e.g., the memory 130 of FIG. 1), and/or an interface (e.g., the interface 177 of FIG. 1). The processor may include, for example, one or more of a central processing unit, an application processor, a graphic processing unit (GPU), an application processor, a sensor processor, or a communication processor.

The memory may include, for example, volatile memory (e.g., the volatile memory 132 of FIG. 1) or nonvolatile memory (e.g., the nonvolatile memory 134 of FIG. 1). The interface may include, for example, a high definition multimedia interface (HDMI), a universal serial bus (USB), an SD card interface, and/or an audio interface. For example, the interface may electrically or physically connect the electronic device 300 to an external electronic device, and may include a USB connector, an SD card/MMC connector, or an audio connector.

The battery 370 (e.g., the battery 189 of FIG. 1), which is a device for supplying power to at least one component of the electronic device 300, may include, for example, a non-rechargeable primary battery, a rechargeable secondary battery, or a fuel cell. For example, at least a portion of the battery 370 may be disposed on substantially the same plane as the PCB 380. The battery 370 may be integrally disposed inside the electronic device 200 or may be detachably disposed from the electronic device 200.

The first antenna 350 may be disposed between the display 220 and the support member 360. The first antenna 350 may include, for example, a near field communication (NFC) antenna, a wireless charging antenna, and/or a magnetic secure transmission (MST) antenna. For example, the first antenna 350 may perform short-range communication with an external device, wirelessly transmit and receive power required for charging, and transmit a short-range communication signal or a magnetic-based signal including payment data. In another embodiment, an antenna structure may be formed by some or a combination of the frame 310 and/or the support member 360.

The second antenna 355 may be disposed between the PCB 380 and the rear plate 393. The second antenna 355 may include, for example, a near field communication (NFC) antenna, a wireless charging antenna, and/or a magnetic secure transmission (MST) antenna. For example, the second antenna 355 may perform short-range communication with an external device, wirelessly transmit and receive power required for charging, and transmit a short-range communication signal or a magnetic-based signal including payment data. In another embodiment, an antenna structure may be formed by some or a combination of the frame 310 and/or the rear plate 393.

The sealing member 390 may be disposed between the frame 310 and the rear plate 393. The sealing member 390 may be configured to block moisture and foreign matter flowing into a space surrounded by the frame 310 and the rear plate 393 from the outside. The electronic devices 200 and 300 may be referred to as a wearable device in terms of being worn on a part (e.g., wrist) of the user's body.

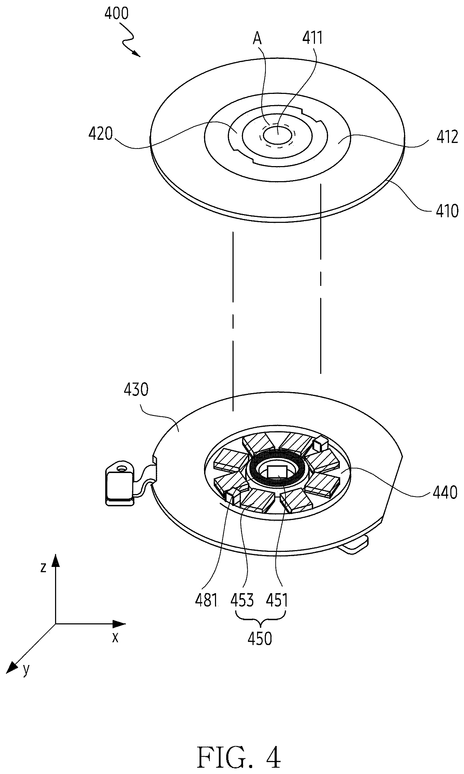

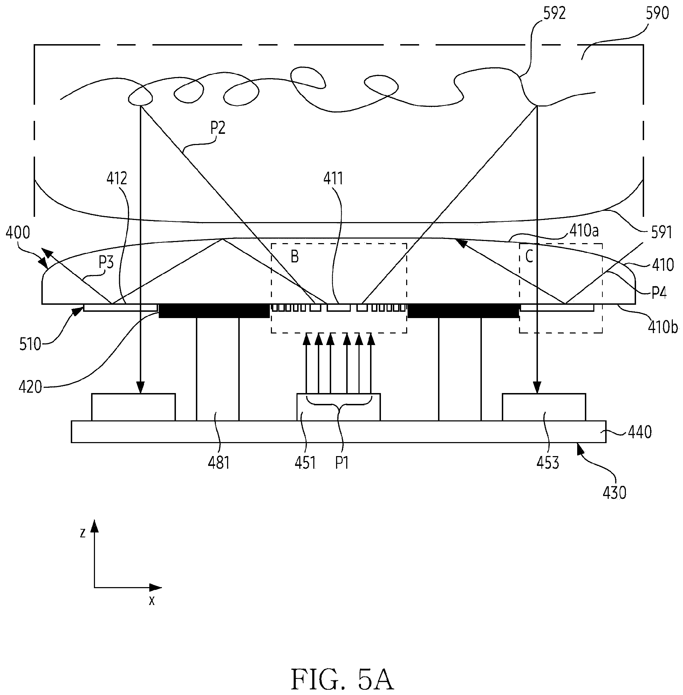

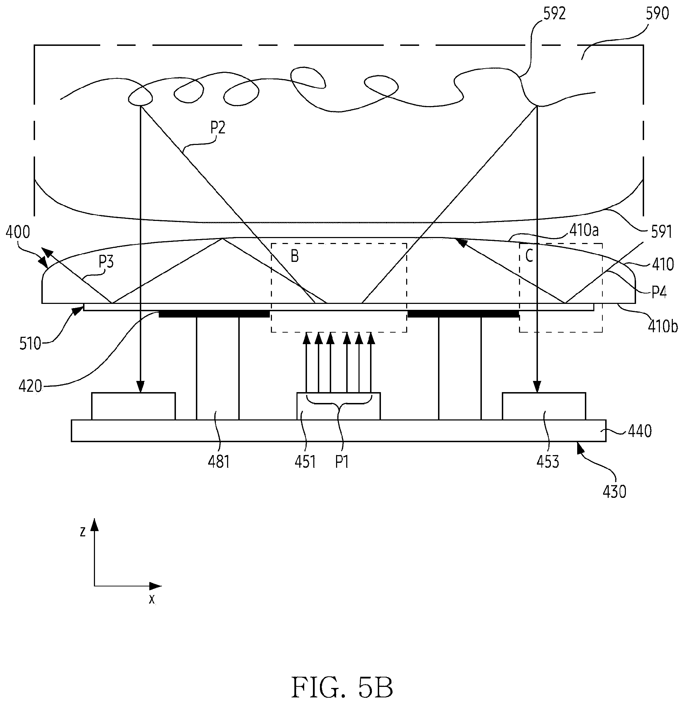

FIG. 4 is an exploded perspective view illustrating a cover and an optical module of an electronic device according to an embodiment. FIGS. 5A and 5B are schematic views illustrating an electronic device including a cover and an optical module in a state in which an electronic device is in contact with an external object, according to an embodiment.

Referring to FIG. 4, an electronic device 400 (e.g., the electronic device 101 of FIG. 1, the electronic device 200 of FIG. 2A, or the electronic device 300 of FIG. 3) may include a cover 410 (e.g., the rear plate 207 of FIG. 2A or the rear plate 393 of FIG. 3) and an optical module 430. The cover 410 may form at least a portion of an exterior of the electronic device 400. The cover 410 may at least partially include a transparent portion. The cover 410 may protect electronic components disposed inside the electronic device 400. The cover 410 may transmit light emitted from the optical module 430 disposed under the cover 410 (e.g., in -z axis direction) to the outside of the electronic device 400. The cover 410 may transmit some of the light transmitted from the outside of the electronic device 400 to the inside of the electronic device 400. The cover 410 may include a first optical coating 411 and a second optical coating 412. The first optical coating 411 may be disposed in a region of the cover 410 at least partially overlapping a light-emitting portion 451. The second optical coating 412 may be disposed in a region of the cover 410 at least partially overlapping light-receiving portions 453. The first optical coating 411 and the second optical coating 412 may be configured to reflect light outside a designated angle range. The optical coatings 411 and 412 may be formed of a plurality of layers. For example, one layer of layers of the optical coatings 411 and 412 may have a refractive index different from that of other layers in contact with the layer. The optical coatings 411 and 412 may include SiO₂ or TiO₂.

According to an embodiment, the first optical coating 411 and the second optical coating 412 may be configured to at least partially transmit light incident within a designated angle range. The first optical coating 411 and the second optical coating 412 may be formed of substantially the same material. The first optical coating 411 and the second optical coating 412 may be anti-reflective coatings. The cover 410 may include glass, sapphire, reinforced plastic, or polymer material. The cover 410 may include a light-blocking material 420. The light-blocking material 420 may be disposed on a surface of the cover 410. The light-blocking material 420 may be disposed between the first optical coating 411 and the second optical coating 412. The light-blocking material 420 may reduce unintended light from being transmitted to the light-receiving portions 453. For example, it may prevent light emitted from the light-emitting portion 451 from being transmitted to the light-receiving portions 453 through the coating layer (e.g., the coating layer 510 of FIG. 5A). According to an embodiment, the light-blocking material 420 is represented as being disposed inside the coating layer 510, but the light-blocking material 420 may be disposed on the coating layer 510.

According to an embodiment, the optical module 430 may include a printed circuit board 440 (e.g., the PCB 380 of FIG. 3), a light-emitting portion 451, and light-receiving portions 453. The printed circuit board 440 may be electrically connected to components (e.g., electronic components) required for driving the electronic device 400. For example, the printed circuit board 440 may be electrically connected to electronic components disposed on the printed circuit board 440. According to an embodiment, the printed circuit board 440 may be disposed inside the electronic device 400 (e.g., inside the housing). The printed circuit board 440 may be spaced apart from the cover 410.

According to an embodiment, the light-emitting portion 451 and the light-receiving portions 453 may be disposed on a printed circuit board 440. The light-emitting portion 451 and the light-receiving portions 453 may be electrically connected to a processor (e.g., the processor 120 of FIG. 1) through the printed circuit board 440. The processor 120 may be configured to emit light to the outside through the light-emitting portion 451. The processor 120 may obtain information about an external environment based on light received through the light-receiving portions 453. For example, the processor 120 may emit light toward an external object through the light-emitting portion 451. The processor 120 may obtain information related to the external object based on some of light that is reflected by the external object and at least partially transmitted to the light-receiving portions 453. The information may be biometric information obtained through light that is reflected from blood vessels or bones in the user's body and transmitted to light-receiving portions 453. According to an embodiment, the light-emitting portion 451 and the light-receiving portions 453 may be a photoplethysmogram (PPG) sensor configured to obtain biometric information from a body sensor of the user. The PPG sensor may be configured to emit light into the user's body and receive light reflected from the user's body to detect a change in blood flow within microvasculature. The PPG sensor may be configured to emit light into the user's body and receive light reflected from the user's body to detect a change in blood flow within microvasculature. For example, a volume of microvasculature of the user's body may change because the amount of a blood flow inside the microvasculature of the user changes due to a periodic contraction or relaxation of the heart. The amount of light emitted from the PPG sensor and transmitted into the user's body and absorbed by the user's blood vessels may change according to a change in the amount of the blood flow in a portion of the user's body. The PPG sensor may be configured to obtain biometric data related to the user's body, based on identifying the intensity of light reflected from the user's body. For example, the biometric data may include at least one of a change in the user's heart rate over a designated period, oxygen saturation, or blood pressure. According to an embodiment, the optical module 430 may include a laser, a light emitting diode (LED), or an organic light emitting diode (OLED) configured to obtain biometric information from the user's body. For example, light emitted from the light-emitting portion 451 may penetrate a portion of the user's body and reach a designated substance (e.g., glucose in blood or alcohol molecules in blood) of a body part (e.g., the body part 590 of FIG. 5A or FIG. 5B) of the user. After a wavelength and/or intensity of light reaching the designated material is changed, light emitted from the light-emitting portion 451 may be reflected from a portion (e.g., tissue 592 of FIG. 5A or FIG. 5B) of the user's body part 590, and the reflected light may be transmitted at least partially to the light-receiving portions 453. The optical module 430 may obtain other biometric data about the user's body based on receiving the light reflected from a portion (e.g., blood vessels) of the user's body. For example, the other biometric data may mean oxygen saturation, blood sugar level, or blood alcohol concentration in the user's body.

According to an embodiment, the light-emitting portion 451 may be disposed on a region of the printed circuit board 440. The light-emitting portion 451 may include a laser, an LED, or an OLED. The light-receiving portions 453 may include a plurality of light sensors. The light-receiving portions 453 may be photodiodes that convert light into electrical energy. The light-receiving portions 453 may surround an edge of the light-emitting portion 451. For example, a plurality of photodiodes may be disposed on the printed circuit board 440 along a perimeter of the light-emitting portion 451.

According to an embodiment, the optical module 430 may include a partition wall 481. The partition wall 481 may be disposed between the light-emitting portion 451 and the light-receiving portions 453. The partition wall 481 may separate a space where the light-emitting portion 451 and the light-receiving portions 453 are disposed. The partition wall 481 may surround the light-emitting portion 451. For example, the partition wall 481 may form a closed curve that surrounds an edge of the light-emitting portion 451. The partition wall 481 may prevent light emitted from the light-emitting portion 451 from being transmitted to the light-receiving portions 453 inside the electronic device 400.

Referring to FIG. 5A, the electronic device 400 may be worn on a body part 590 of the user. The electronic device 400 may be a wearable device. For example, the electronic device 400 may be a watch-type electronic device worn on a wrist, which is a body part 590 of the user. The cover 410 of the electronic device 400 may be in contact with the body part 590 of the user. For example, the cover 410 may be in contact with a surface of a skin 591.

According to an embodiment, light emitted from the optical module 430 may be transmitted to the body part 590 through the cover 410. The cover 410 may include a transparent portion capable of transmitting light. A surface 410a of the cover 410 may contact the body part 590 of the user. The surface 410a of the cover 410 may be a portion of an outer surface of the electronic device 400. Another surface 410b opposite to the surface 410a of the cover 410 may face the optical module 430.

According to an embodiment, the coating layer 510 may be disposed on the other surface 410b of the cover 410. The coating layer 510 may be an anti-reflective coating. The coating layer 510 may be formed of a plurality of layers. The coating layer 510 may be deposited on the other surface 410b of the cover 410. The coating layer 510 may enable a portion of lights P1 transmitted through the cover 410 to transmit to the outside of the electronic device 400, and limit reflection to the inside of the electronic device 400. The coating layer 510 may limit a remaining portion of the light transmitted through the cover 410 from being transmitted into the electronic device 400 and reflect it to the outside of the cover 410.

According to one embodiment, the coating layer 510 may be thinner than an anti-reflective film. For example, the coating layer 510 may be about 0.1 µm to 3 µm. The coating layer 510 may be formed by coating the other surface 410b of the cover 410 with an anti-reflection material. For example, the coating layer 510 may be deposited on the other surface 410b of the cover 410 or a coating liquid may be applied thereto. A thinly formed coating layer 510 may reduce light emitted from the light-emitting portion 451 and transmitted into the coating layer 510 from being transmitted to the light-receiving portions 453. For example, as a thickness of the coating layer 510 becomes thinner, a degree to which an optical path of light from the light-emitting portion 451 is spaced apart from an optical axis may be reduced. An optical path, which is slightly spaced apart from the optical axis, may not face the light-receiving portions 453, but may be reflected from the surface 410a of the cover 410 and proceed toward the body part 590 of the user.

According to an embodiment, the coating layer 510 may include a first optical coating 411 and a second optical coating 412. The coating layer 510 may provide an optical coating to provide an appropriate optical path for light transmitted from the light-emitting portion 451 or to increase the accuracy of sensing data of the light-receiving portions 453. The first optical coating 411 may be disposed on the light-emitting portion 451. The second optical coating 412 may be disposed on the light-receiving portions 453. The first optical coating 411 may include an optical pattern therein. For example, the first optical coating 411 may include a plurality of slits. An optical pattern formed on the first optical coating 411 by the plurality of slits may diffract lights P1 transmitted from the light-emitting portion 451 and focus the lights P1 at a designated location. The first optical coating 411 may focus the lights P1 at the designated location, thereby reducing attenuation of the light emitted from the light-emitting portion 451 and transmitting it to the tissue 592 of the body part 590.

According to an embodiment, the optical module 430 may include the light-emitting portion 451 and the light-receiving portions 453 disposed on the printed circuit board 440. The light-emitting portion 451 may emit light toward the first optical coating 411. The light-receiving portions 453 may receive light transmitted through the second optical coating 412. For example, the light-emitting portion 451 may emit the lights P1 toward the first optical coating 411.

According to an embodiment, partial lights P2 among the lights P1 transmitted to the first optical coating 411 may be transmitted into the inside of the body part 590 and transmitted to internal tissues (e.g., blood vessels or bones) 592. Remaining partial lights P3, excluding the partial lights P2 among the partial light P1 transmitted to the first optical coating 411, may be reflected in a portion other than the tissue. For example, the remaining partial lights P3 may be light reflected from inside the body part 590 without being transmitted to the surface 410a of the cover 410, a surface of the skin 591, or the tissue 592.

According to an embodiment, the second optical coating 412 may be configured to transmit the partial lights P2 transmitted from the tissue 592 among the lights P1 emitted from the light-emitting portion 451 and reflect the remaining partial lights P3 among the lights P1 emitted from the light-emitting portion 451. The second optical coating 412 may increase the accuracy of the light-receiving portions 453 by transmitting the partial lights P2 from the tissue 592 to the light-receiving portions 453 and reflecting the remaining lights P3.

According to an embodiment, the second optical coating 412 may be configured to prevent light transmitted from the light-emitting portion 451 from being reflected inside the optical coating and transmitted to the light-receiving portions 453. For example, the second optical coating 412 may be formed to be thin. For example, the second optical coating 412 may be about 0.1 µm to 1 µm or less. The coating layer 510 including the first optical coating 411 and the second optical coating 412 may be about 0.1 µm to 1 µm or less. The coating layer 510 may be formed to be thinner than attaching a separate optical film by being deposited on the cover 410.

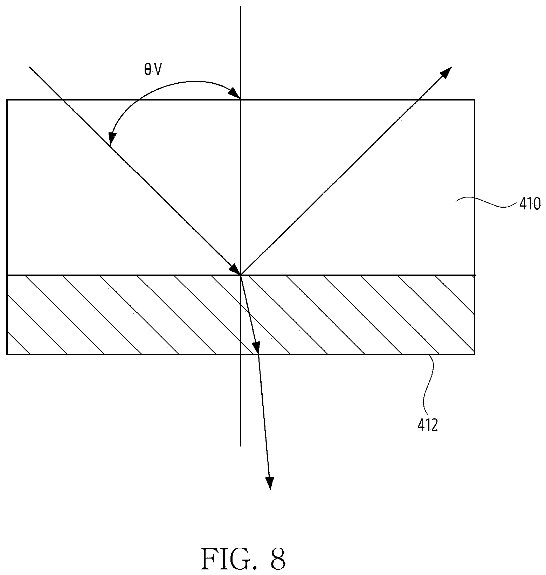

Referring to FIG. 5B, the coating layer 510 may be configured to transmit a portion of light incident on the coating layer 510 toward the light-receiving portion 453 and reflect a remaining portion. A partial light with an angle smaller than a predetermined angle among lights incident on the coating layer 510 may be transmitted to the light-receiving portions 453 through the coating layer. A partial light with an incident angle greater than a predetermined angle among the lights incident on the coating layer 510 may be reflected by the coating layer 510 and not transmitted to the light-receiving portions 453. The coating layer 510 may be configured to transmit a portion of the lights emitted from the light-emitting portion 451 and reflect a remaining portion. Among the light emitted to the coating layer 510, a partial light with an incident angle smaller than a predetermined angle may be transmitted into the skin by passing through the coating layer 510. A light path according to an incident angle of light incident on the coating layer 510 is described in FIG. 8. The light-blocking material 420 may be disposed between a region of the coating layer 510 overlapping the light-emitting portion 451 and a region of the coating layer 510 overlapping the light-receiving portion 453. The light-blocking material 420 may be disposed on the coating layer 510. However, it is not limited thereto. According to an embodiment, as illustrated in FIG. 5A, the coating layer 510 may include a first optical coating disposed in a region overlapping the light-emitting portion 451 and a second optical coating disposed in a region overlapping the light-receiving portions 453. The second optical coating may be spaced apart from the first optical coating. The first optical coating may not include a pattern, unlike FIG. 5A. The light-blocking material 420 may be disposed between the first optical coating and the second optical coating.

According to an embodiment, an optical module 430 including the light-emitting portion 451 and the light-receiving portions 453 may include a PPG sensor. The optical module 430 may identify a heart rate, stress index, and blood oxygen saturation of a user wearing the electronic device 400, based on the lights P2 transmitted to the light-receiving portions 453.

According to an embodiment, the second optical coating 412 may prevent external light P4 from being transmitted to the light-receiving portions 453. For example, the external light P4 transmitted through the cover 410 to a space between the user's skin 591 and the electronic device 400 may not be transmitted to the inside of the electronic device 400 by the second optical coating 412.

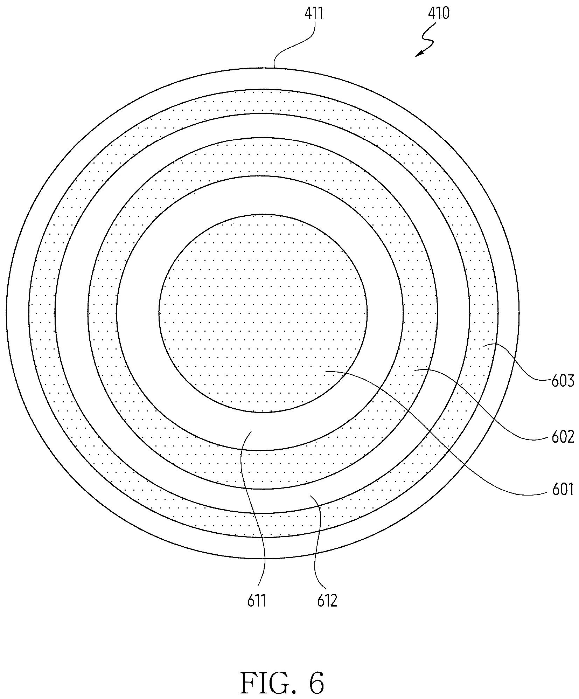

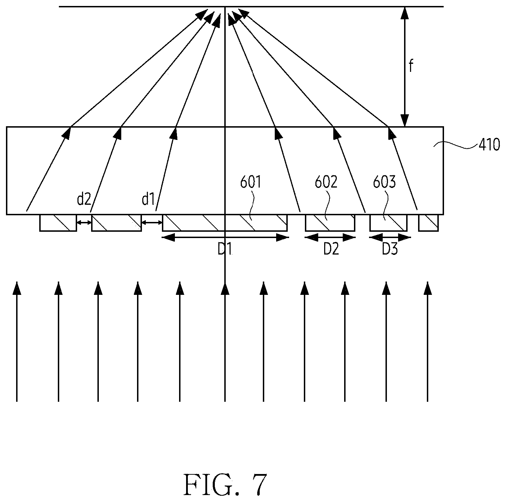

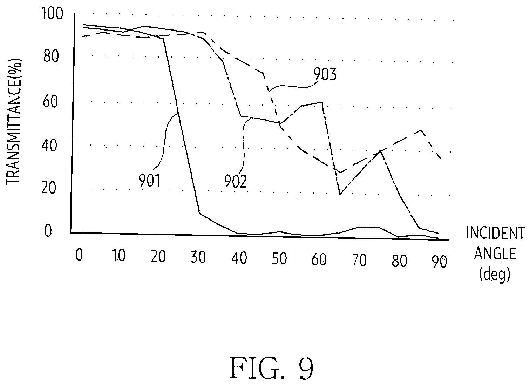

According to an embodiment, the coating layer 510 may be configured to reduce attenuation of the lights P1 emitted from the light-emitting portion 451 and transmit it to the body part 590 of the user. For example, the first optical coating 411 of the coating layer 510 may minimize the attenuation of light and focus the light, by including a pattern. The pattern of the first optical coating 411 will be described through FIGS. 6 and 7. The second optical coating 412 of the coating layer 510 may adjust transmittance of the light according to the incident angle. Filtering of the light transmitted to the second optical coating 412 will be described through FIGS. 8 and 9.

FIG. 6 is a diagram illustrating a pattern of a first optical coating disposed in a region A of FIG. 4 of the cover according to an embodiment. FIG. 7 is an enlarged view of a region B of FIG. 5A according to an embodiment.

Referring to FIGS. 6 and 7, a first optical coating 411 coated on a cover 410 may include a first pattern 601, a second pattern 602, and a third pattern 603. The first pattern 601 may be disposed within the first optical coating 411. The first pattern 601 may be a circular coating disposed on the cover 410 and having a diameter D1. The second pattern 602 may have a first width D2. The first width D2 may be smaller than the diameter D1 of the first pattern 601. The second pattern 602 may be spaced apart from an outer periphery of the first pattern 601 by a first distance d1. The second pattern 602 may surround an edge of the first pattern 601. The third pattern 603 may have a second width D3. The second width D3 may be smaller than the first width D2 of the second pattern 602. The third pattern 603 may be spaced apart from an outer periphery of the second pattern 602 by a second distance d2. The first distance d1 may be longer than the second distance d2. The third pattern 603 may surround an edge of the second pattern 602.

According to an embodiment, the first optical coating 411 may include an uncoated first region 611 having a width of the first distance d1 and an uncoated second region 612 having a width of the second distance d2. The first region 611 and the second region 612 may be masking regions. For example, while the first optical coating 411 is deposited on the cover 410, the first region 611 and the second region 612 may be masked. The first optical coating 411 may be deposited except for the first region 611 and the second region 612. For another example, the first region 611 and the second region 612 may be regions where the first optical coating 411 is etched. When manufacturing the first optical coating 411, the manufacturing cost may be reduced and the yield may be improved by selectively removing unnecessary portions (e.g., the first region 611 and the second region 612) of the first optical coating 411 or selectively performing a deposition process.

According to an embodiment, the first optical coating 411 may transmit light transmitted from a light-emitting portion (e.g., the light-emitting portion 451 of FIGS. 4 to 5B) to a body part (e.g., the body part 590 of FIGS. 5A or 5B) through the first region 611 and the second region 612. The first optical coating 411 may block, through the first pattern 601, the second pattern 602, and the third pattern 603, light from being transmitted from the light-emitting portion 451 to the body part 590. The first region 611 and the second region 612 of the first optical coating 411 may operate as a slit configured to diffract light. Lights P1 passing through the first region 611 and the second region 612 of the first optical coating 411 constructively interfere with each other, so light may be focused at a location spaced apart by a focal distance f.

According to an embodiment, the focal distance f of the lights P1 emitted from the light-emitting portion 451 may be determined based on the shape of the patterns 601, 602, and 603 and a distance between the patterns 601, 602, and 603. According to an embodiment, as the focal distance f becomes closer, the diameter D1 of the first pattern 601, the first width D2 of the second pattern 602, the second width D3 of the third pattern 603, the first distance d1, and the second distance d2 may become smaller. For example, as the focal distance f becomes closer, sizes of the patterns 601, 602, and 603 become smaller, and an interval between the patterns 601, 602, and 603 decreases, so the number of patterns may increase. For example, as the focal distance f decreases, the number of patterns may increase, and as the focal distance f increases, the number of patterns may decrease.

According to an embodiment, a coating layer (e.g., the coating layer 510 of FIG. 5A or FIG. 5B) may be configured to prevent some of the lights P1 transmitted from the light-emitting portion 451 from being reflected on an inner surface of the first optical coating 411 and transmitted to the light-receiving portions (e.g., the light-receiving portions 453 of FIGS. 4 to 5B). For example, a thickness of the first optical coating 411 may be about 0.1 µm or more and 1 µm or less. As the thickness of the first optical coating 411 is formed to be thin, the reflection of the lights P1 on the inner surface of the coating layer 510 and its transmission to the light-receiving portions 453 may be reduced.

According to the above-described embodiment, the coating layer 510 may focus the light emitted from the light-emitting portion 451 at a designated location through the pattern of the first optical coating 411. The coating layer 510 may reduce attenuation of the light by focusing the light emitted from the light-emitting portion 451 at the designated location.

FIG. 8 is an enlarged view of a region C of FIG. 5A or FIG. 5B, according to an embodiment. FIG. 9 is a graph illustrating a relationship between an incident angle and transmittance of light incident on an optical coating according to an embodiment.

Referring to FIGS. 8 and 9, a second optical coating 412 may be attached to a cover 410. The second optical coating 412 may be configured to transmit a portion of light incident on the second optical coating 412 and reflect a remaining portion. Among lights incident on the second optical coating 412, light having an incident angle ΘV smaller than a predetermined angle may be transmitted through the second optical coating 412 to light-receiving portions 453 (e.g., the light-receiving portions 453 of FIG. 5A). Among the lights incident on the second optical coating 412, light having an incident angle ΘV larger than the predetermined angle may be reflected by the second optical coating 412 and may not be transmitted to the light-receiving portions 453.

Referring back to FIG. 5A, lights facing the light-receiving portions 453 may be partial lights P2 from among lights P1 transmitted to the first optical coating 411, remaining lights P3 excluding the partial lights P2 from the lights P1 transmitted to the first optical coating 411, and an external light P4. The partial lights P2 may be light reflected by tissue 592 located inside a body part 590 (e.g., the body part 590 of FIG. 5A). The partial lights P2 are transmitted into and reflected inside the body part 590, so an incident angle ΘV formed with a direction perpendicular to the second optical coating 412 disposed on the light-receiving portions 453 may be relatively smaller than that of the remaining lights P3 and the external light P4. For example, an incident angle of light reflected from the first optical coating 411 to a surface 410a of the cover 410 and transmitted to the second optical coating 412 may be greater than that of the partial light P2. Since the external light P4 passes between the skin 591 and the electronic device 400 and is transmitted to the cover 410, the incident angle ΘV formed with the direction perpendicular to the second optical coating 412 may be relatively greater than that of other lights.

According to an embodiment, the second optical coating 412 may be configured to transmit only the incident angle ΘV less than or equal to the designated angle, so the remaining lights P3 and external light P4 may not be transmitted. The second optical coating 412 may prevent the remaining lights P3 and the external light P4, which are noise, from being transmitted to the light-receiving portions 453, by transmitting only lights reflected by the tissue 592 of the body part 590.

Referring to FIG. 9, a graph indicates transmittance of a second optical coating (e.g., the second optical coating 412 of FIG. 4, FIG. 5A, and FIG. 8) for incident light, according to an embodiment. The value of X-axis may be an incident angle of light transmitted to the second optical coating 412. The value of Y-axis may be transmittance of the light for the second optical coating 412.

Graph 901 may indicate transmittance of green light, graph 902 may indicate transmittance of red light, and graph 903 may indicate transmittance of infrared light. The green light may be light used to obtain heart rate or stress index. The red light and the infrared light may be light used to obtain data related to oxygen saturation or blood glucose.

Looking at the graph 901, green light shows transmittance of about 90% or more when an incident angle is about 25 degrees or less. Looking at the graph 902, red light shows transmittance of about 90% or more when an incident angle is about 30 degrees or less. Looking at the graph 903, infrared light shows transmittance of about 90% or more when an incident angle is about 35 degrees or less.

According to an embodiment, the second optical coating 412 may be configured to transmit, among lights (e.g., the lights P2, P3, and P4 of FIG. 5A), lights (e.g., the partial lights P2 of FIG. 5A) for which an angle between a line segment perpendicular to the second optical coating and an optical path is about 20 degrees or less.

According to the above-described embodiment, the coating layer 510 may detect a user's bio-signal with relatively high accuracy by not transmitting lights corresponding to noise to the light-receiving portions 453 through the second optical coating 412.

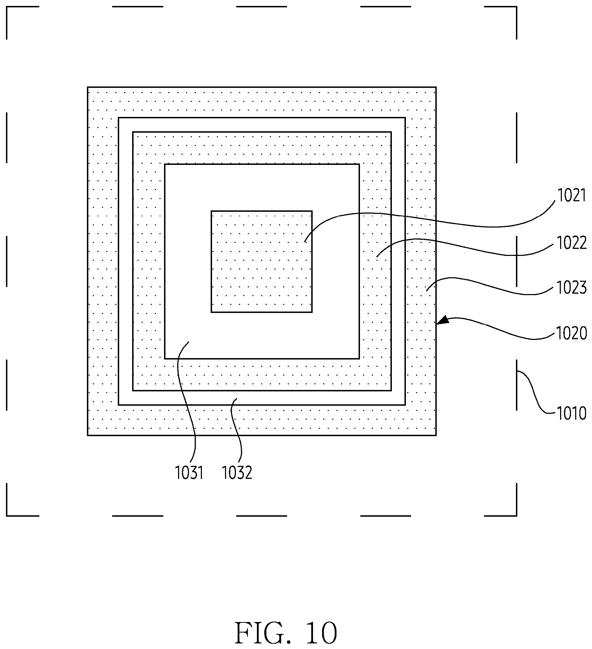

FIG. 10 is a diagram illustrating an exemplary pattern of a first optical coating according to an embodiment.

Referring to FIG. 10, a coating layer 1010 may include a first optical coating 1020 disposed on a light-emitting portion (e.g., the light-emitting portion 451 of FIG. 4). The first optical coating 1020 may include a first pattern 1021, a second pattern 1022, and a third pattern 1023. The first pattern 1021 and the second pattern 1022 may be spaced apart from each other, and the second pattern 1022 and the third pattern 1023 may be spaced apart from each other. A first uncoated region 1031 may be disposed between the first pattern 1021 and the second pattern 1022. A second uncoated region 1032 may be disposed between the second pattern 1022 and the third pattern 1023. A width of the first uncoated region 1031 may be wider than a width of the second uncoated region 1032. The coating layer 1010 may be formed substantially identically to the coating layer 510 of FIG. 5A, except for the shape. For example, the first pattern 1021 may be formed in a polygonal shape. The first pattern 1021 may have a shape corresponding to an outer shape of an electronic device (e.g., the electronic device 400 of FIG. 4). For example, the first pattern 1021 or the coating layer 1010 may be formed in a rectangular shape. When a shape of the electronic device 400 or a cover (e.g., the cover 410 of FIG. 4) of the electronic device 400 is a rectangle, the first pattern 1021 or the coating layer 1010 may be formed in a rectangular shape corresponding to the cover. In addition, coating layers or patterns in various shapes (e.g., a rectangle with rounded corners or a chamfered rectangle) may be provided. For focusing light emitted from a light-emitting portion (e.g., the light-emitting portion 451 of FIG. 4), a width of patterns may become narrower as moving away from the center of the coating layer 1010. An interval between patterns may become narrower as moving toward the outer periphery of the coating layer 1010.

According to the above-described embodiment, the coating layer 1010 may provide patterns 1021, 1022, and 1023 for focusing light transmitted from the light-emitting portion 451 by including uncoated regions 1031 and 1032. Since a separate film or lens for focusing light may be omitted, a thickness of the electronic device 400 may be reduced, and efficiency of an internal space of the electronic device 400 may be increased.

According to the above-described embodiment, an electronic device (e.g., the electronic device 101 of FIG. 1, the electronic device 200 of FIG. 2A, the electronic device 300 of FIG. 3, or the electronic device 400 of FIG. 4) may include a cover (e.g., the cover 410 of FIG. 4). The cover may form at least a portion of an exterior of the electronic device and may include a transparent portion. The electronic device may further include an optical module (e.g., the optical module 430 of FIG. 4). The optical module may include a light-emitting portion (e.g., the light-emitting portion 451 of FIG. 4), spaced apart from the cover and configured to transmit first light to the outside through the transparent portion and at least one light-receiving portion (e.g., the light-receiving portions 453 of FIG. 4) spaced apart from the light-emitting portion, configured to receive second lights through the transparent portion, and surrounding an outer periphery of the light-emitting portion. The electronic device may include a first optical coating (e.g., the first optical coating 411 of FIG. 4 or the first optical coating 1020 of FIG. 10). The optical coating may be disposed on a surface of the transparent portion facing the light-emitting portion. The electronic device may include a second optical coating (e.g., the second optical coating 412 of FIG. 4). The second optical coating may be spaced apart from the first optical coating and disposed on a surface of the transparent portion facing the at least one light-receiving portion. The first optical coating may include concentric patterns with respect to a location overlapping the light-emitting portion.

According to the above-described embodiment, the electronic device may provide light that penetrates a part of the user's body through an optical coating disposed on a rear surface of the cover. The electronic device may reduce, through a second optical coating disposed on the light-receiving portion, light corresponding to noise transmitted from the outside from being transmitted to the light-receiving portion. The electronic device may include a first optical coating instead of a lens configured to focus light emitted from the light-emitting portion. The electronic device may include a second optical coating to reduce noise transmitted to the light-receiving portion. The electronic device may provide a coating layer instead of a film or lens, thereby reducing a thickness of the electronic device and improving space efficiency.

According to an embodiment, the patterns may be configured to focus the first light on a focal distance spaced apart from the cover by diffracting first light emitted from the light-emitting portion.

According to the above-described embodiment, light emitted from the light-emitting portion may be focused on a designated point through patterns formed at the first optical coating. The electronic device may omit other components for focusing light of the light-emitting portion by including the patterns.

According to an embodiment, the patterns may include a first pattern, a second pattern spaced apart from an outer periphery of the first pattern by a first distance, surrounding an edge of the first pattern, and having a first width, and a third pattern spaced apart from an outer periphery of the second pattern by a second distance, surrounding an edge of the second pattern, and having a second width. The first distance may be longer than the second distance. The first width may be longer than the second width. The first width may be smaller than the width of the first pattern.

According to the above-described embodiment, light emitted from the light-emitting portion may be focused on a designated point through patterns formed at the first optical coating. The electronic device may omit other components for focusing light of the light-emitting portion by including the patterns.

According to an embodiment, a focal distance of first light emitted from the light-emitting portion may be determined based on the first distance and the second distance.

According to the above-described embodiment, the first optical coating may adjust the focal distance based on a spaced distance between the patterns. The electronic device may adjust a distance between patterns forming the first optical coating, based on a distance to tissue of a body part of a user.

According to an embodiment, the first optical coating may be configured to prevent a portion of the first light transmitted from the light-emitting portion from being transmitted to the light-receiving portion by being reflected at an inner surface of the first optical coating.

According to the above-described embodiment, the electronic device may reduce transmission of light emitted from the light-emitting portion to the light-receiving portion through the optical coating before being transmitted to body tissue.

According to an embodiment, a thickness of the first optical coating and the second optical coating may be 0.1 micrometer or more and 1 micrometer or less.

According to the above-described embodiment, the electronic device may reduce transmission of light from the light-emitting portion to the light-receiving portion through the optical coating, by thinly forming a thickness of the optical coating.

According to an embodiment, the electronic device may further include a light-blocking material (e.g., the light-blocking material 420 of FIG. 4) disposed between the first optical coating and the second optical coating.

According to the above-described embodiment, the electronic device may prevent light from being transmitted from the light-emitting portion to the light-receiving portion through the light-blocking material, in a state where it is difficult to reduce the thickness of the optical coating.

According to an embodiment, the second optical coating may be configured to a portion of the second light for which an angle between a line segment perpendicular to the second optical coating and an optical path is 20 degrees or less.

According to the above-described embodiment, the electronic device may apply an anti-reflection coating to reduce noise transmitted to the light-receiving portion. The second optical coating may be configured to block light larger than a designated incident angle and transmit light smaller than the designated incident angle, thereby reducing transmission of external light or light reflected from the skin to the light-receiving portion.

According to an embodiment, the electronic device may further include a partition wall (e.g., the partition wall 481 of FIG. 4) disposed between the light-emitting portion and the light-receiving portion. According to an embodiment, the partition wall may surround the light-emitting portion.

According to the above-described embodiment, the electronic device may prevent light emitted from the light-emitting portion from being transmitted to the light-receiving portion inside the electronic device, by including a partition wall disposed between the light-emitting portion and the light-receiving portion.

According to an embodiment, the at least one light-receiving portion may include a plurality of light-receiving portions spaced apart from each other.

According to an embodiment, the plurality of light-receiving portions may be disposed along a perimeter of the light-emitting portion.

According to the above-described embodiment, the electronic device may transmit light, which is transmitted from the light-emitting portion and reflected from tissue of a body part, to the light-receiving portion, by disposing the light-receiving portions around the light-emitting portion. The electronic device may obtain a user's biometric signal based on the light transmitted through the light-receiving portion.

According to an embodiment, the electronic device may further include a printed circuit board (e.g., the printed circuit board 440 of FIG. 4) on which the light-emitting portion and the at least one light-receiving portion are disposed.

According to the above-described embodiment, the electronic device may include a printed circuit board on which the light-emitting portion and the light-receiving portion are disposed, and the printed circuit board may be electrically connected to a processor (e.g., the processor 120 of FIG. 1). The processor may be configured to obtain information related to a user's biometric signal through the light-emitting portion and the light-receiving portions.

According to an embodiment, the first lights may penetrate, through the cover, an external object in contact with the cover, and the second lights, which are a portion of the first light reflected inside the external object, may be transmitted toward the light-receiving portion.

According to an embodiment, the optical sensor may include a PPG sensor, the light-emitting portion may include one of an LED, an OLED or a laser, and the light-receiving portion may include a photodiode.

According to the above-described embodiment, the electronic device may obtain biometric information based on information on light received from an external object (e.g., a body part of the user) by including an optical module including a light-emitting portion and light-receiving portions.

According to an embodiment, the first optical coating and the second optical coating may include a plurality of layers.

According to an embodiment, the first optical coating and the second optical coating may include at least one of SiO₂ or TiO₂.

According to the above-described embodiment, the electronic device may control a refractive index based on the optical coatings formed in a plurality of layers. By controlling the refractive index, the transmittance of light according to an incident angle of the second optical coating disposed on the light-receiving portion may be controlled.

According to an embodiment, an electronic device (e.g., the electronic device 101 of FIG. 1, the electronic device 200 of FIG. 2A, the electronic device 300 of FIG. 3, or the electronic device 400 of FIG. 4) may include a cover (e.g., the cover 410 of FIG. 4). The cover may form at least a portion of an exterior of the electronic device and may include a transparent portion. The electronic device may further include an optical module (e.g., the optical module 430 of FIG. 4). The optical module may include a light-emitting portion (e.g., the light-emitting portion 451 of FIG. 4) spaced from the cover and configured to transmit first light to the outside through the transparent portion, at least one light-receiving portion (e.g., the light-receiving portions 453 of FIG. 4) spaced apart from the light-emitting portion, configured to receive second light through the transparent portion, and surrounding an outer periphery of the light-emitting portion, and a partition wall (e.g., the partition wall 481 of FIG. 4) disposed between the light-emitting portion and the light-receiving portion. The electronic device may further include a first optical coating (e.g., the first optical coating 411 of FIG. 4 or the first optical coating 1020 of FIG. 10). According to an embodiment, the first optical coating may be disposed on a surface of the transparent portion facing the light-emitting portion. The electronic device may further include a second optical coating (e.g., the second optical coating 412 of FIG. 4). The second optical coating may be spaced apart from the first optical coating, and may be disposed on a surface of the transparent portion facing the at least one light-receiving portion. The electronic device may further include a light-blocking material (e.g., the light-blocking material 420 of FIG. 4). The light-blocking material may be disposed between the first optical coating and the second optical coating. According to an embodiment, the first optical coating may include a first pattern (e.g., the first pattern 601 of FIG. 6), a second pattern (e.g., the second pattern 602 of FIG. 6) spaced apart from an outer periphery of the first pattern by a first distance, surrounding an edge of the first pattern, and having a first width, and a third pattern (e.g., the third pattern 603 of FIG. 6) spaced apart from an outer periphery of the second pattern by a second distance, surrounding an edge of the second pattern, and having a second width.

According to the above-described embodiment, the electronic device may provide light penetrating a body part of the user through an optical coating disposed on a rear surface of a cover. The electronic device may reduce, through a second optical coating disposed on the light-receiving portion, light corresponding to noise transmitted from the outside from being transmitted to the light-receiving portion. The electronic device may include a first optical coating instead of a lens configured to focus light emitted from the light-emitting portion. The electronic device may include a second optical coating to reduce noise transmitted to the light-receiving portion. The electronic device may reduce a thickness of the electronic device and improve space efficiency, by providing a coating layer instead of a film or lens.

According to an embodiment, the first pattern, the second pattern and the third pattern may be configured to focus the first light on a focal distance spaced apart from the cover by diffracting the first light emitted from the light-emitting portion.

According to the above-described embodiment, light emitted from the light-emitting portion may be focused on a designated point through patterns formed at the first optical coating. The electronic device may omit other components for focusing light of the light-emitting portion by including the patterns.

According to an embodiment, the at least one light-receiving portion may include a plurality of light-receiving portions spaced apart from each other.

According to an embodiment, the plurality of light-receiving portions may be disposed along a perimeter of the light-emitting portion.

According to the above-described embodiment, the electronic device may transmit light, which is transmitted from the light-emitting portion and reflected from tissue of a body part, to the light-receiving portion, by disposing the light-receiving portions around the light-emitting portion. The electronic device may obtain a user's biometric signal based on the light transmitted through the light-receiving portion.

According to an embodiment, the electronic device may further include a printed circuit board (e.g., the printed circuit board 440 of FIG. 4) on which the light-emitting portion and the at least one light-receiving portion are disposed.

According to the above-described embodiment, the electronic device may include a printed circuit board on which the light-emitting portion and the light-receiving portion are disposed, and the printed circuit board may be electrically connected to a processor (e.g., the processor 120 of FIG. 1). The processor may be configured to obtain information related to a user's biometric signal through the light-emitting portion and the light-receiving portions.

According to an embodiment, the optical sensor may include a PPG sensor. According to an embodiment, the light-emitting portion may include one of an LED, an OLED, or a laser.

According to an embodiment, the light-receiving portion may include a photodiode.

According to the above-described embodiment, the electronic device may obtain biometric information based on information on light received from an external object (e.g., a body part of the user) by including an optical module including a light-emitting portion and a light-receiving portions.

According to an embodiment, the electronic device may include a cover forming at least a portion of an exterior of the electronic device and including a transparent portion. The electronic device may include an optical module that includes a light-emitting portion, at least one light-receiving portion, and a partition wall. The light-emitting portion may be configured to be spaced apart from the cover and transmit first light to the outside through the transparent portion. The at least one light-receiving portion may be configured to be spaced apart from the light-emitting portion and receive second light through the transparent portion, and may surround an outer periphery of the light-emitting portion. The partition wall may be disposed between the light-emitting portion and the at least one light-receiving portion. The electronic device may include a light-blocking material. The light-blocking material may include an optical coating disposed on a surface of the transparent portion facing the light-emitting portion and the light-receiving portion, and a light-blocking material disposed between a region of the optical coating overlapping the light-emitting portion and a region of the optical coating overlapping the light-receiving portion.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (101; 200; 300; 400) comprising:
a cover (410), forming at least a portion of an exterior of the electronic device (101; 200; 300; 400), including a transparent portion;
an optical module (430) including:
a light-emitting portion (451), spaced apart from the cover (410), configured to transmit first light to an outside through the transparent portion, and
at least one light-receiving portion (453), spaced apart from the light-emitting portion (451), configured to receive second light through the transparent portion, and surrounding an outer periphery of the light-emitting portion (451);
a first optical coating (411; 1020) disposed on a surface of the transparent portion facing the light-emitting portion (451); and
a second optical coating (412), spaced apart from the first optical coating (411; 1020), disposed on the surface of the transparent portion facing the at least one light-receiving portion (453),
wherein the first optical coating (411; 1020) includes patterns (611; 612; 613; 1021; 1022; 1023) concentric with a location overlapping the light-emitting portion (451).

2. The electronic device (101; 200; 300; 400) of claim 1,
wherein the patterns (611; 612; 613; 1021; 1022; 1023) are configured to focus the first light on a focal distance (f) spaced apart from the cover (410) by diffracting the first light emitted from the light-emitting portion (451).

3. The electronic device (101; 200; 300; 400) of claim 1 or claim 2,
wherein the patterns (611; 612; 613; 1021; 1022; 1023) include:
a first pattern (611; 1021);
a second pattern (612; 1022), spaced apart from an outer periphery of the first pattern (611; 1021) by a first distance (d1), surrounding an edge of the first pattern (611; 1021), and having a first width (D2); and
a third pattern (613; 1023), spaced apart from an outer periphery of the second pattern (612; 1022) by a second distance (d2), surrounding an edge of the second pattern (612; 1022), and having a second width (D3),
wherein the first distance (d1) is longer than the second distance (d2), and
wherein the first width (D2) is longer than the second width (D3).

4. The electronic device (101; 200; 300; 400) of any one of claims 1 to 3,
wherein a focal distance (f) of the first light emitted from the light-emitting portion (451) is determined based on the first distance (d1) and the second distance (d2).

5. The electronic device (101; 200; 300; 400) of any one of claims 1 to 4,
wherein the first optical coating (411; 1020) is configured to prevent a portion of the first light transmitted from the light-emitting portion (451) from being transmitted to the light-receiving portion (453) by being reflected at an inner surface of the first optical coating (411; 1020).

6. The electronic device (101; 200; 300; 400) of any one of claims 1 to 5,
wherein a thickness of the first optical coating (411; 1020) is 0.1 micrometer or more and 1 micrometer or less.

7. The electronic device (101; 200; 300; 400) of any one of claims 1 to 6, further comprising a light-blocking material disposed between the first optical coating (411; 1020) and the second optical coating (412).

8. The electronic device (101; 200; 300; 400) of any one of claims 1 to 7,
wherein the second optical coating (412) is configured to transmit a portion of the second light for which an angle between a line segment perpendicular to the second optical coating (412) and an optical path is 20 degrees or less.

9. The electronic device (101; 200; 300; 400) of any one of claims 1 to 8,
further comprising a partition wall (481) disposed between the light-emitting portion (451) and the light-receiving portion (453),
wherein the partition wall (481) surrounds the light-emitting portion (451).

10. The electronic device (101; 200; 300; 400) of any one of claims 1 to 9,
wherein the at least one light-receiving portion (453) includes a plurality of light-receiving portions (453) spaced apart from each other, and
wherein the plurality of light-receiving portions (453) are disposed along a perimeter of the light-emitting portion (451).

11. The electronic device (101; 200; 300; 400) of any one of claims 1 to 10, further comprising a printed circuit board (440) on which the light-emitting portion (451) and the at least one light-receiving portion (453) are disposed.

12. The electronic device (101; 200; 300; 400) of any one of claims 1 to 11,
wherein the first light transmits, through the cover (410), an external object in contact with the cover (410), and
wherein the second light, which is a portion of the first light reflected inside the external object, is transmitted toward the at least one light-receiving portion (453).

13. The electronic device (101; 200; 300; 400) of any one of claims 1 to 12,
wherein the optical module (430) includes a photoplethysmography (PPG) sensor,
wherein the light-emitting portion (451) includes at least one of a light emitting diode (LED), an organic light emitting diode (OLED), or a laser, and
wherein the light-receiving portion (453) includes a photodiode.

14. The electronic device (101; 200; 300; 400) of any one of claims 1 to 13,
wherein the first optical coating (411; 1020) and the second optical coating (412) include a plurality of layers.

15. The electronic device (101; 200; 300; 400) of any one of claims 1 to 14,
wherein the first optical coating (411; 1020) and the second optical coating (412) include at least one of silicon dioxide (SiO₂) or titanium dioxide (TiO₂).
